# EUROPÄISCHE PATENTSCHRIFT

(11) **EP 1 090 584 B1**
(45) Veröffentlichungstag und Bekanntmachung des Hinweises auf die Patenterteilung: **28.09.2011**
(21) Anmeldenummer: 00117504.1
(22) Anmeldetag: 12.08.2000
(51) Int. Cl.: A61B 5/15

(54) **Blutlanzettenvorrichtung zur Entnahme von Blut für Diagnosezwecke**
Blood lancet device for obtaining blood samples for diagnosis purposes
Dispositif pour le prélèvement du sang par lancette à des fins de diagnostique

(30) Priorität: 09.10.1999 DE 19948759
(43) Veröffentlichungstag der Anmeldung: 11.04.2001
(73) Patentinhaber: Roche Diagnostics GmbH, 68305 Mannheim (DE)
(72) Erfinder: Kuhr, Hans-Jürgen, Dr., 68219 Mannheim (DE); Argauer, Herbert, 92712 Pirk (DE)
(74) Vertreter: Pfeifer, Hans-Peter

(56) Entgegenhaltungen:
- US-A- 5 035 704
- US-A- 5 514 152
- US-A- 5 527 334
- US-A- 5 545 174
- US-A- 5 938 679

## Beschreibung

Die Erfindung betrifft eine Blutlanzettenvorrichtung zur Entnahme von Blut für Diagnosezwecke, welche einen in einem Gehäuse entlang einem vorbestimmten gerade Einstichweg beweglichen Lanzettenhalter zur Halterung der Lanzette und eine Lanzettenführung, von der der Lanzettenhalter auf dem vorbestimmten Einstichweg geführt wird, aufweist.

Um einen Blutstropfen zu gewinnen, wird bei solchen Blutlanzettenvorrichtungen eine die Austrittsöffnung umgebende Kontaktfläche gegen die Haut (insbesondere an der Fingerspitze oder einem Ohrläppchen) gedrückt und ein Einstichvorgang ausgelöst, bei dem der Lanzettenhalter und die darin gehaltene Lanzette von einem in der Blutlanzettenvorrichtung vorhandenen Lanzettenantrieb mit hoher Geschwindigkeit entlang dem vorbestimmten Einstichweg in Einstichrichtung bewegt wird, bis ihre Spitze aus der Austrittsöffnung austritt, um die zur Gewinnung des Blutstropfens erforderliche Wunde zu erzeugen. Danach wird der Lanzettenhalter durch den Lanzettenantrieb in eine Position zurückgeführt, bei der sich die Spitze der Lanzette in dem Gehäuse befindet.

Derartige Lanzettenvorrichtungen sind in zahlreichen Ausführungsformen bekannt. Meist wird der Lanzettenantrieb von einem einfachen Mechanismus gebildet, bei dem der Lanzettenhalter durch eine Linearfeder direkt vorgetrieben wird und im Punkt der größten Austrittsweite der Lanzettenspitze auf einen Anschlag auftrifft. Dadurch wir die Lanzette ruckartig gestoppt und durch die Federwirkung in die Ausgangslage zurückgeführt. Eine solche Blutlanzettenvorrichtung ist einfach herstellbar, in der Funktion jedoch nicht optimal, insbesondere weil der bei dem Einstich verursachte Schmerz relativ groß ist.

Die Reduktion des durch die Erzeugung der Wunde verursachten Schmerzes ist jedoch von großer medizinischer Bedeutung. Dies gilt insbesondere für Diabetiker, die ihren Blutzuckerspiegel häufig und regelmäßig kontrollieren, um die notwendigen Insulininjektionen an den Bedarf (der von der Nahrungsaufnahme, der körperlichen Aktivität und anderen Faktoren abhängt) anzupassen und dadurch den Blutzuckerspiegel möglichst ständig innerhalb bestimmter Sollgrenzen zu halten. Dies ist für die Gesundheit solcher Patienten und für die Vermeidung schwerwiegender Spätschäden, wie beispielsweise Erblindung, von allergrößter Bedeutung. In umfassenden Untersuchungen wurde festgestellt, daß durch enge Kontrolle des Blutzuckerspiegels schwerwiegende Langfristschäden des Diabetes Mellitus dramatisch vermindert werden können.

Eine schmerzarme Blutentnahme erlauben Blutlanzettengeräte, bei denen die Entspannungsbewegung der Antriebsfeder mittels eines drehbaren Antriebsrotors in die Einstichbewegung umgesetzt wird. Dadurch wird die mit dem Auftreffen des Lanzettenhalters auf einen Anschlag verbundene Vibration vermieden.

Eine Blutlanzettenvorrichtung mit einem solchen Rotorantrieb wird in dem US-Patent 4,924,879 beschrieben. Dabei wird der Rotor durch eine koaxiale Spiralfeder angetrieben. Die Drehbewegung des Rotors wird über ein Pleuelantrieb in die erforderliche Linearbewegung der Lanzette umgesetzt.

Bei der in dem US-Patent 5,318,584 beschriebenen Lanzettenvorrichtung dreht sich der Antriebsrotor um eine zu der Einstichrichtung parallele Drehachse. Auch in diesem Fall wird er von einer koaxialen Spiralfeder angetrieben. Die Umwandlung der Rotationsbewegung in die erforderliche Linearbewegung des Lanzettenhalters erfolgt mittels eines Drehschiebegetriebes, das vorzugsweise mittels einer Kurvensteuerung realisiert ist. Dabei weist der Rotor eine Ausnehmung auf, in die ein passender Steuerzapfen des Lanzettenhalters eingreift. Durch diese Konstruktion wird ein sehr gutes Einstichverhalten mit geringen Vibrationen und einer präzise reproduzierbaren Einstichtiefe erreicht. Dadurch ist der mit der Erzeugung der Wunde verbundene Schmerz sehr gering. Nachteilig ist jedoch, daß die Konstruktion das präzise Zusammenwirken relativ kompliziert geformter Bauteile erfordert. Die Herstellung ist deswegen aufwendig und kostspielig. Eine zuverlässige Funktion setzt eine präzise Fertigung voraus.

Aus der EP 0 458 451 A1 ist eine für einen einmaligen Gebrauch konzipierte Lanzettenvorrichtung mit einem Lanzettenantrieb bekannt, der zwei schwenkbar gekoppelte Arme umfaßt, die ein Kniegelenk bilden: Bei der Einstichbewegung werden die beiden Arme aus einer zueinander gebeugten Position über eine vollständig gestreckte Einstichposition in eine, in umgekehrter Richtung gebeugte Stellung geschwenkt. Bei dieser Schwenkbewegung wird die Lanzette zunächst vorgeschoben, bis die beiden Arme die vollständig gestreckte Einstichposition erreichen, und anschließend wieder zurückgezögen. Die Schwenkbewegung wird über eine Blattfeder induziert, die beim Auslösen einer Einstichbewegung auf den Vortriebsabschnitt des Schwenkarms schlägt.
Aufgrund der Geometrie des Gehäuses und der fehlenden Möglichkeit die Lanzettenvorrichtung zu spannen, kann der auf den Vortriebsabschnitt folgende Abschnitt des Schwenkarms nicht mit der Blattfeder in Kontakt kommen.

Der Erfindung liegt auf dieser Basis die Aufgabe zugrunde, eine Blutlanzettenvorrichtung zur Verfügung zu stellen, bei der ein präzises und vibrationsarmes Einstichverhalten mit reduziertem Herstellungsäufwand und demzufolge verminderten Kosten erreicht wird.

Die Aufgabe wird bei einer Blutlanzettenvorrichtung der eingangs erläuterten Art mit Rotorantrieb des Lanzettenhalters dadurch erreicht, daß der Antriebsrotor eine nach radial außen gerichtete Druckfläche aufweist, die in variierendem Achsabstand derartig um die Rotationsachse herum verläuft, daß sie einen Scheitelpunkt mit maximalem Achsabstand und einen dem Scheitelpunkt entgegen der Drehrichtung folgenden Vortriebsabschnitt mit entgegen der Drehrichtung abnehmendem Achsabstand aufweist, die Antriebsfeder so ausgebildet ist, daß sie in einem Vortriebs-Drehwinkelbereich des Antriebsrotors über ein Druckelement einen Druck mit einer radial in Richtung auf die Rotationsachse gerichteten Komponente auf den Vortriebsabschnitt der Druckfläche ausübt, so daß der Antriebsrotor durch den Druck der Antriebsfeder auf die Druckfläche in dem Vortriebsabschnitt in Drehrichtung angetrieben wird, und der Antriebsrotor über einen abtriebsseitigen Kopplungsmechanismus derartig mit dem Lanzettenhalter gekoppelt ist, daß die Spitze der Lanzette aus der Austrittsöffnung austritt, während sich der Antriebsrotor in dem Vortriebs-Drehwinkelbereich befindet.

Der Antriebsrotor mit nach radial außen (weg von der Rotationsachse) gerichteter Druckfläche läßt sich einfach als Kunststoffteil herstellen. Die Kraftübertragung von der Antriebsfeder in die Rotationsbewegung des Antriebsrotors (die nachfolgend als "antriebsseitiger Kopplungsmechanismus des Lanzettenantriebs" bezeichnet wird) wird durch die Formgebung des Vortriebsabschnitts der Druckfläche bestimmt. Durch unterschiedliche Gestaltungen der Druckfläche können demzufolge verschiedene gewünschte Bewegungscharakteristiken des Antriebsrotors (Rotationsgeschwindigkeit in Abhängigkeit von dem Drehwinkel) eingestellt werden. Als vorteilhaft hat sich eine Formgebung erwiesen, bei der der Vortriebsabschnitt mindestens teilweise konvex gekrümmt ist.

Zum Spannen muß der Antriebsrotor in eine Position gebracht werden, bei der das Druckelement in der Nähe des Scheitelpunktes auf der Druckfläche aufliegt. Dies kann mit unterschiedlichen Spannmechanismen bewirkt werden. Bevorzugt erfolgt nicht nur der Antrieb der Einstichbewegung, sondern auch das Spannen des Lanzettenantriebs durch ein Zusammenwirken der Feder und der Druckfläche, wobei die Druckfläche einen Spannabschnitt mit entgegen der Drehrichtung zunehmendem Achsabstand aufweist. Auch der Spannabschnitt ist vorzugsweise wenigstens zum Teil konvex gekrümmt.

Gemäß einer weiteren bevorzugten Ausführungsform wird der abtriebsseitige Kopplungsmechanismus, durch den die Drehbewegung des Antriebsrotors in die Einstich- und Rückführungsbewegung des Lanzettenhalters übertragen wird, durch eine gemeinsam mit dem Antriebsrotor drehbare, eine Steuerkurve bildende Ausnehmung gebildet, in die ein mit dem Lanzettenhalter verbundener Steuerzapfen eingreift. Bei dieser Konstruktion kann durch die Gestaltung der Steuerkurve auch der abtriebsseitige Kopplungsmechanismus entsprechend dem gewünschten Einstichverhalten variiert werden. Damit ergeben sich umfassende Möglichkeiten, das Geschwindigkeitsprofil der Einstichbewegung (Abhängigkeit der momentanen Geschwindigkeit von der Position des Lanzettenhalters) im Hinblick auf einen möglichst geringen Schmerz zu optimieren. Eine besonders einfache Gestaltung wird erreicht, wenn die die Steuerkurve bildende Ausnehmung in dem Antriebsrotor ausgebildet ist.

Die Erfindung wird nachfolgend anhand von in den Figuren dargestellten Ausführungsbeispielen näher erläutert. Die darin beschriebenen Besonderheiten können einzeln oder in Kombination miteinander eingesetzt werden, um bevorzugte Ausgestaltungen der Erfindung zu schaffen. Es zeigen:
- Fig. 1: einen Längsschnitt durch eine erfindungsgemäße Blutlanzettenvorrichtung während des Einsetzens einer Lanzette in die Lanzettenhalterung;
- Fig. 2: einen Längsschnitt durch die Vorrichtung nach Fig. 1 in der Ruheposition des Lanzettenan- triebs;
- Fig. 3: einen Längsschnitt durch die Vorrichtung nach Fig. 1 in der gespannten Position des Lanzet- tenantriebs;
- Fig. 4: einen Längsschnitt durch eine alternative Aus- führungsform einer Blutlanzettenvorrichtung in der Ruheposition;
- Fig. 5: einen Längsschnitt durch die Vorrichtung nach Fig. 4 in der gespannten Position des Lanzet- tenantriebs;
- Fig. 6: eine Seitenansicht des Antriebsrotors der Vor- richtung von Fig. 4 und
- Fig. 7: eine Seitenansicht des Spannhebels der Vorrich- tung von Fig. 4.

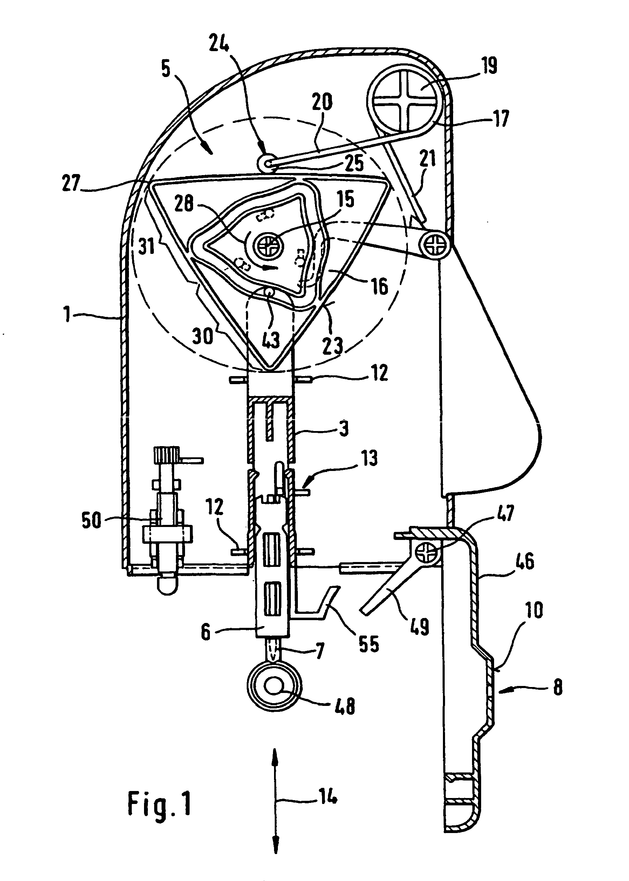

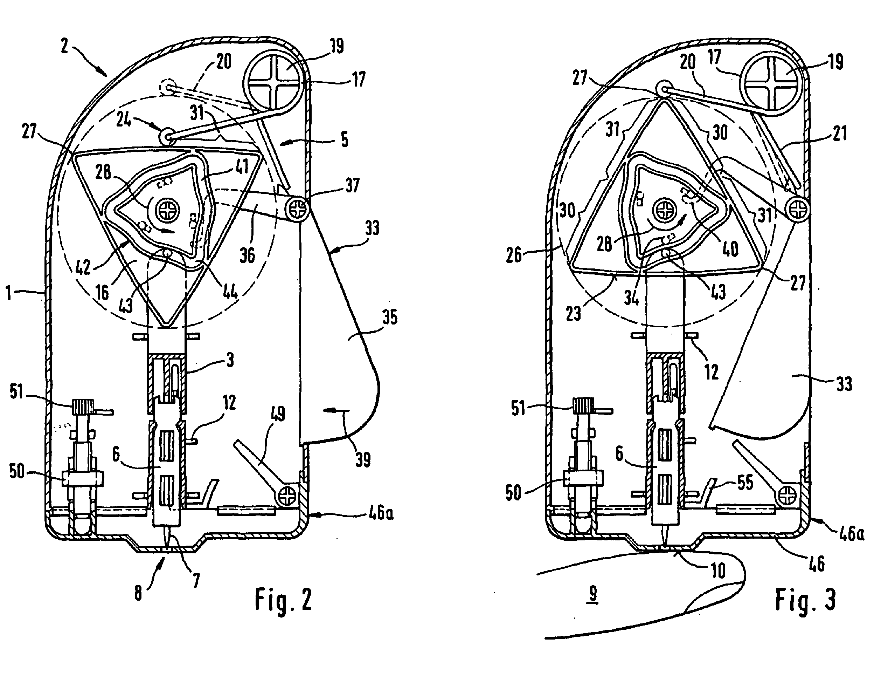

In dem Gehäuse 1 der in den Fig. 1 bis 3 dargestellten Blutlanzettenvorrichtung 2 wird ein Lanzettenhalter 3 von einem insgesamt mit 5 bezeichneten Lanzettenantrieb derartig angetrieben, daß eine in dem Lanzettenhalter 3 befestigte Lanzette 6 eine Einstichbewegung durchführt, bei der ihre Spitze 7 aus einer Austrittsöffnung 8 des Gehäuses 1 austritt, um in einem an der Austrittsöffnung 8 anliegenden Körperteil 9 (Fig. 3) eine Wunde zu erzeugen. Die zum Anlegen an das Körperteil 9 vorgesehene die Austrittsöffnung umgebende Fläche wird als Kontaktfläche 10 bezeichnet. Die Einstich- und Rückführbewegung des Lanzettenhalters 3 wird von einer im dargestellten Fall durch Kunststoffstege 12 gebildeten Lanzettenführung 13 auf einem linearen geraden Einstichweg präzise geführt, so daß praktisch keine Vibrationen mit Bewegungskomponenten senkrecht zu der in Fig. 1 durch den Pfeil 14 symbolisierten Einstichrichtung auftreten können.

Der Lanzettenantrieb 5 besteht im wesentlichen aus einem um eine Achse 15 rotierenden Antriebsrotor 16 und einer Antriebsfeder 17, die im dargestellten Fall als um einen Fixierungspfosten 19 gewickelte Drehfeder mit zwei Schenkeln 20, 21 ausgebildet ist.

Der Antriebsrotor 16 weist eine nach radial außen gerichtete Druckfläche 23 auf, die in variierendem Achsabstand um die Rotationsachse 15 herum verläuft. Ein erster Schenkel 20 der Antriebsfeder 17 übt über ein Druckelement 24 einen Druck mit einer radial in Richtung auf die Rotationsachse 15 gerichteten Komponente auf die Druckfläche 23 aus. Diese Druckübertragung sollte möglichst reibungsfrei sein. Deswegen ist das Druckelement 24 als am Ende des ersten Schenkels 20 befestigtes, auf der Druckfläche 23 laufendes Rad 25 ausgebildet. Der zweite Schenkel 21 ist außerhalb des Drehbereiches 26 des Antriebsrotors 16 fixiert.

Der Antriebsrotor 16 ist 3-strahligsymmetrisch mit der Achse 15 als Symmetriezentrum, so daß sich seine Formelemente in einem Drehwinkelabstand von 120° wiederholen. Die Druckfläche 23 ist in der Zeichenebene der Figuren 1 bis 3, die mit der Rotationsebene des Antriebsrotors 16 übereinstimmt, näherungsweise dreieckig. Ihre (leicht abgerundeten) Ecken bilden Scheitelpunkte 27, die den maximalen Abstand der Druckfläche 23 von der Rotationsachse 15 markieren. An die Scheitelpunkte 27 schließt sich entgegen der durch den Pfeil 28 bezeichneten Drehrichtung des Antriebsrotors 16 ein Abschnitt mit abnehmendem Achsabstand an, der als Vortriebsabschnitt 30 bezeichnet wird. Den Scheitelpunkten 27 (bezogen auf die Drehrichtung 28) voraus gehen jeweils Abschnitte der Druckfläche 23, deren Achsabstand in Drehrichtung abnimmt (also entgegen der Drehrichtung zunimmt) und die als Spannabschnitte 31 bezeichnet werden. Insgesamt ist die Druckfläche 23 zwischen den Scheitelpunkten 27 in der Rotationsebene leicht konvex gekrümmt.

Um den Lanzettenantrieb 5 zu spannen, muß der Lanzettenrotor 16 von der in Fig. 2 gezeigten Ruheposition in Drehrichtung 28 gedreht und dadurch in die in Fig. 3 dargestellte gespannte Position gebracht werden. Dies kann beispielsweise mittels eines elektrischen Rotorantriebs geschehen. Bevorzugt ist jedoch eine mechanische Spannvorrichtung. Sie weist im dargestellten Fall einen Spannhebel 33 auf, der ein Drehmoment in Drehrichtung 28 auf ein an dem Antriebsrotor vorgesehenes Widerlagerteil 34, das im dargestellten Fall durch zylindrische, von der Stirnfläche des Antriebsrotors 16 vorstehende Stifte gebildet wird, ausübt. Der Spannhebel 33 besteht aus einer im Ruhezustand (Fig. 2) seitlich aus dem Gehäuse 1 herausragenden Betätigungstaste 35 und einem in dem Gehäuse 1 verlaufenden Schwenkarm 36. Zwischen der Betätigungstaste 35 und dem Schwenkarm 36 befindet sich ein Schwenklager 37, dessen Achse zu der Rotationsachse 15 des Antriebsrotors 16 parallel verläuft. Zum Spannen des Lanzettenantriebs 5 wird die Betätigungstaste 35 in Richtung des Pfeiles 39 in das Gehäuse gedrückt. Dadurch schwenkt der Schwenkarm 36 nach oben und ein am Ende des Schwenkarms 36 vorgesehener Haken 40 erfaßt eines der Widerlagerteile 34, so daß der Rotor 16 in Drehrichtung 28 gedreht wird. Dabei fährt das Druckelement 24 einen Spannabschnitt 31 der Druckfläche 23 ab. Die Entfernung des Druckelementes 24 von der Rotationsachse 15 nimmt zu, wobei die Antriebsfeder 17 nach und nach gespannt wird, bis sich ihr erster Schenkel 20 in der (in Fig. 2 gestrichelt dargestellten) Position maximaler Entfernung von der Rotationsachse 15 befindet. Wenn der Lanzettenantrieb 5 vollständig gespannt ist, befindet sich der Antriebsrotor 16 in der in Fig. 3 dargestellten Position, bei der das Druckelement 24 gegen einen Scheitelpunkt 27 der Druckfläche 23 drückt.

Sobald der Lanzettenrotor 16 in Drehrichtung 28 weitergedreht wird, gelangt das Druckelement 24 in den Vortriebsabschnitt 30 der Druckfläche 23. Damit beginnt ein Drehwinkelbereich der Bewegung des Antriebsrotors, in dem der Druck der Antriebsfeder 17 (der im wesentlichen auf die Rotationsachse 15 gerichtet ist, zumindest eine Komponente in dieser Richtung hat) in ein Drehmoment umgesetzt wird, das den Antriebsrotor 16 in Drehrichtung 28 antreibt. Dieser Drehwinkelbereich, in dem das Druckelement 24 in Kontakt mit dem Vortriebsabschnitt 30 steht, wird als Vortriebs-Drehwinkelbereich bezeichnet.

Die Antriebsfeder 17 mit dem Druckelement 24 und die Druckfläche 23 bilden insgesamt einen antriebsseitigen Kopplungsmechanismus, durch den eine Drehung des Antriebsrotors 16 erzeugt wird, die in dem Vortriebs-Drehwinkelbereich rasch und selbsttätig (ohne daß in dieser Bewegungsphase eine Einwirkung des Benutzers möglich oder erforderlich ist) abläuft. Um diese Drehbewegung in die erforderliche Linearbewegung des Lanzettenhalters 3 mit der Lanzette 6 umzusetzen, ist ein abtriebsseitiger Kopplungsmechanismus erforderlich, der auf unterschiedliche Weise realisiert sein kann. Beispielsweise ist ein Pleuelmechanismus, wie bei dem US-Patent 4,924,879, geeignet.

Bevorzugt ist jedoch die in den Figuren dargestellte Ausführungsform, bei der eine gemeinsam mit dem Antriebsrotor 16 drehbare, vorzugsweise in dem Antriebsrotor 16 selbst ausgebildete, von einer Ausnehmung 41 gebildete Steuerkurve 42 vorgesehen ist, in die ein mit dem Lanzettenhalter 3 verbundener Steuerzapfen 43 so eingreift, daß mindestens ein Teil der Einstich- und Rückführbewegung durch die Drehung der Steuerkurve 42 relativ zu dem Steuerzapfen bestimmt wird. Dabei fährt der Steuerzapfen die Steuerkurve 42 ab. Die maximale Auslenkung des Lanzettenhalters 3 in Einstichrichtung wird durch einen unteren Umkehrpunkt 44 der Steuerkurve 42 bestimmt, der so positioniert ist, daß er von dem Steuerzapfen 43 innerhalb des Vortriebs-Drehwinkelbereiches der Rotorbewegung abgefahren wird. Der Steuerzapfen ist vorzugsweise unmittelbar an dem Lanzettenhalter 3 (nämlich an dessen von der Lanzettenspitze 7 abgewandtem Ende) angebracht.

Um das Einsetzen der Lanzette 6 in den Lanzettenhalter 3 zu erleichtern, ist an dem unteren Ende des Gehäuses 1 ein unteres Abschlußteil 46 vorgesehen, das eine längliche Form mit parallel zu der Rotorebene (d.h. in der Zeichenebene der Figuren 1 bis 3) verlaufender Hauptachse hat. Es ist einseitig an einer seiner Schmalseiten 46a schwenkbar (über ein Scharnier 47 mit parallel zur Rotorachse verlaufender Schwenkachse) an dem übrigen Gehäuse angelenkt. Zum Einsetzen der Lanzette wird das untere Ende des Gehäuses 1 durch Wegschwenken des unteren Abschlußteiles 46 (Fig. 1) geöffnet, so daß der Lanzettenhalter 3 gut zugänglich ist. Die Lanzette 6 wird an einer ihre Spitze 7 abdeckenden Schutzkappe 48 ergriffen und in die Lanzettenhalterung 3 eingeschoben. Nähere Einzelheiten über geeignete Lanzettenhalterungen sind dem US-Patent 5,318,584 und den darin zitierten Druckschriften zu entnehmen.

Das untere Abschlußteil 46 ist mit einem Schwenkarm 49 gekoppelt, der beim Öffnen des Abschlußteils 46 mit einem Ausleger 55 kollidiert. Der Ausleger 55 wirkt mit dem Lanzettenhalter 3 derartig zusammen, daß eine darin eingesetzte Lanzette infolge der Kollision mit dem Schwenkarm 49 beim Öffnen des Abschlußteils 46 automatisch aus der Lanzettenhalterung 3 ausgeworfen wird.

Zur Einstellung der Einstichtiefe ist an der dem Scharnier 47 gegenüberliegenden Schmalseite des unteren Abschlußteils 46 eine Verstellvorrichtung 50 vorgesehen, durch die der Abstand (in Einstichrichtung) zwischen dem unteren Abschlußteil 46 und dem übrigen Gehäuse 1 eingestellt werden kann. Je größer dieser Abstand ist, desto kleiner wird (bei unverändertem Bewegungsweg des Lanzettenhalters 3) die Einstichtiefe. Zur Einstellung der Verstellvorrichtung 50 ist eine Rändelschraube 51 vorgesehen, die durch eine in der Gehäusewandung vorgesehene nicht dargestellte Ausnehmung von außen her zugänglich ist.

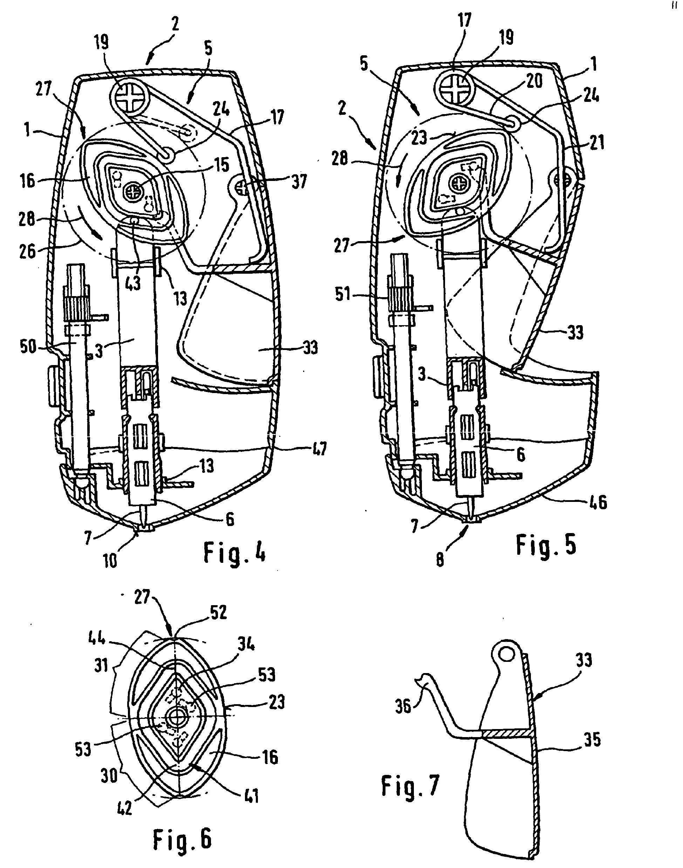

Die in den Figuren 4 und 5 dargestellte Ausführungsform hat im Vergleich zu der in den Figuren 1 bis 3 dargestellten Lanzettenvorrichtung eine schlankere und kompaktere Gehäuseform, stimmt aber in den technischen Merkmalen weitgehend mit dieser überein, wobei funktionsgleiche Bauelemente mit den gleichen Bezugszeichen bezeichnet sind.

Ein Besonderheit besteht darin, daß der Antriebsrotor 16 bei dieser Ausführungsform 2-strahligsymmetrisch ist. Seine Druckfläche 23 hat demzufolge zwei Scheitelpunkte 27 mit jeweils sich daran anschließenden Vortriebs- und Spannabschnitten 30 und 31. Dadurch wird eine kleinere Bauweise möglich, wobei der Rotor 16 besonders kompakt gestaltet werden kann, wenn die Position des Druckelementes 24 gegenüber der Längsachse des Lanzettenhalters 3 seitlich versetzt ist. Dadurch ist es möglich, die Steuerkurve 42 so zu orientieren, daß ihre unteren Umkehrpunkte 44 bezogen auf die Rotationsachse 15 etwa in der gleichen Richtung wie die Scheitelpunkte 27 der Druckfläche 23 orientiert sind, und zugleich die Bedingung einzuhalten, daß der Steuerzapfen 43 den unteren Umkehrpunkt 44 innerhalb des Vortriebs-Drehwinkelbereiches des Antriebsrotors 16 abfährt.

Eine weitere Besonderheit der in den Figuren 4 und 5 dargestellten Ausführungsform wird anhand der in den Figuren 6 und 7 gesondert dargestellten Bauteile deutlich. Während bei den Figuren 1 bis 3 die Druckfläche 23 in der Umgebung der Scheitelpunkte 27 stetig konvex gekrümmt ist, so daß die Spannbewegung bei Betätigung des Spannhebels 33 ohne Stopp in die Vortriebsbewegung des Antriebsrotors 16 übergeht, weist der in Figur 6 dargestellte Antriebsrotor an seinen Scheitelpunkten 27 jeweils eine kleine Vertiefung 52 auf, die so ausgebildet ist, daß das Druckelement 24 darin einrastet. Um aus dieser Position heraus den Übergang in den Vortriebs-Drehwinkelbereich der Rotorbewegung und damit die Einstichbewegung auszulösen, ist ein zusätzlicher Auslösemechanismus vorgesehen, der im dargestellten Fall durch zwei zusätzliche Widerlagerteile 53 des Antriebsrotors 16 und eine besondere Gestaltung des Schwenkarms 36 des Spannhebels 33 realisiert ist. Diese Elemente sind so aufeinander abgestimmt, daß der Eingriff zwischen dem Schwenkarm 36 und dem von diesem betätigten Widerlagerteil 34 in dem Moment unterbrochen wird, in dem das Druckelement 24 in die Vertiefung 52 einrastet. Durch kurzes Loslassen und erneutes Eindrücken der Taste 35 kommt der Schwenkarm 36 in Eingriff mit einem der zusätzlichen Widerlagerteile 53. Dadurch wird die Einstichbewegung ausgelöst.

Zahlreiche weitere Ausgestaltungen der Erfindung sind möglich. Der Antriebsrotor ist bevorzugt N-strahligsymmetrisch (N = 2,3,4). Grundsätzlich ist jedoch auch eine nichtsymmetrische Ausgestaltung möglich. Insbesondere kann ein Antriebsrotor mit nur einem Scheitelpunkt und einer etwa nockenförmigen Gestaltung der Druckfläche 23 verwendet werden. Die Druckfläche 23 ist vorzugsweise eine kontinuierlich stetige, die Achse vollständig umgebende Fläche. Sie kann jedoch auch unterbrochen sein, wenn durch andere konstruktive Maßnahmen sichergestellt ist, daß der Antriebsrotor in einen Zustand gelangt, bei dem sich das Druckelement 24 in der Nachbarschaft eines Scheitelpunktes 27 befindet, an den sich ein Vortriebsabschnitt mit den beschriebenen Merkmalen anschließt. In der Regel ist die Druckfläche 23 eine durch Bewegung einer Geraden im Raum erzeugte Fläche (Regelfläche), wobei die Erzeugende parallel zu der Achse des Antriebsrotors verläuft. Es ist jedoch auch eine gewisse Neigung oder Krümmung der Druckfläche 23 möglich, sofern hierdurch die beschriebenen Funktionen nicht verhindert werden.

Die Erfindung vereint die Vorteile einer einfachen und robusten Mechanik mit einer hochpräzisen Lanzettenführung. Sie kann mit wenigen Bauteilen realisiert und einfach montiert werden. Die meisten Teile bestehen aus Kunststoff und können in großen Stückzahlen kostengünstig - insbesondere durch Spritzen - hergestellt werden. Die Antriebsfeder besteht aus Federstahl und hat eine einfache Geometrie, die ebenfalls eine kostengünstige Produktion erlaubt.

## Patentansprüche

1. Blutlanzettenvorrichtung (2) zur Entnahme von Blut aus einem Körperteil (9) für Diagnosezwecke, umfassend
einen in einem Gehäuse (1) entlang einem vorbestimmten Einstichweg beweglichen Lanzettenhalter (3) zur Halterung einer Lanzette (6),
eine Lanzettenführung (13), von der der Lanzettenhalter (3) auf dem vorbestimmten Einstichweg geführt wird und
einen Lanzettenantrieb (5) mit einem von einer Antriebsfeder (17) angetriebenen in eine Drehrichtung (28) drehbaren Antriebsrotor (16), durch den die Entspannungsbewegung der Antriebsfeder (17) in eine Einstichbewegung umgesetzt wird, bei der die von dem Lanzettenhalter (3) gehaltene Lanzette (6) mit hoher Geschwindigkeit entlang dem vorbestimmten Einstichweg in Einstichrichtung (14) bewegt wird, um die zur Erzeugung einer Wunde in dem Körperteil (9) erforderliche Einstich- und Rückführbewegung durchzuführen,
wobei
der Antriebsrotor eine nach radial außen gerichtete Druckfläche (23) aufweist, die in variierendem Achsabstand derartig um die Rotationsachse (15) herum verläuft, daß sie einen Scheitelpunkt (27) mit maximalem Achsabstand und einen dem Scheitelpunkt (27) entgegen der Drehrichtung (28) folgenden Vortriebsabschnitt (30) mit entgegen der Drehrichtung (28) abnehmendem Achsabstand aufweist,
die Antriebsfeder (17) so ausgebildet ist, daß sie in einem Vortriebs-Drehwinkelbereich des Antriebsrotors (16) über ein Druckelement (24) einen Druck mit einer radial in Richtung auf die Rotationsachse (15) gerichteten Komponente auf den Vortriebsabschnitt (30) der Druckfläche (23) ausübt, so daß der Antriebsrotor (16) durch den Druck der Antriebsfeder (17) auf die Druckfläche in dem Vortriebsabschnitt (30) in Drehrichtung (28) angetrieben wird,
der Antriebsrotor (16) über einen abtriebsseitigen Kopplungsmechanismus derartig mit dem Lanzettenhalter (3) gekoppelt ist, daß die maximale Auslenkung der Lanzette (6) erreicht wird, während sich der Antriebsrotor (16) in dem Vortriebs-Drehwinkelbereich befindet,
und die Druckfläche (23) einen entgegen der Drehrichtung (28) dem Scheitelpunkt (27) vorausgehenden Spannabschnitt (31) mit entgegen der Drehrichtung zunehmendem Achsabstand aufweist, der beim Spannen des Lanzettenantriebs (5) von dem Druckelement(24) abgefahren wird.

2. Blutlanzettenvorrichtung nach Anspruch 1, **dadurch gekennzeichnet, daß** der Vortriebsabschnitt (30) mindestens teilweise konvex gekrümmt ist.

3. Blutlanzettenvorrichtung nach einem der vorhergehenden Ansprüche, **dadurch gekennzeichnet, daß** der Spannabschnitt (31) mindestens teilweise konvex gekrümmt ist.

4. Blutlanzettenvorrichtung nach einem der vorhergehenden Ansprüche, **dadurch gekennzeichnet, daß** die Druckfläche (23) mindestens zwei Scheitelpunkte (27) aufweist.

5. Blutlanzettenvorrichtung nach einem der vorhergehenden Ansprüche, **dadurch gekennzeichnet, daß** die Druckfläche (23) eine kontinuierlich stetige, die Achse vollständig umgebende Fläche ist.

6. Blutlanzettenvorrichtung nach einem der vorhergehenden Ansprüche, **dadurch gekennzeichnet, daß** der abtriebsseitige Kopplungsmechanismus eine gemeinsam mit dem Antrieberotor drehbare, eine Steuerkurve (42) bildende Ausnehmung (41) einschließt, in die ein mit dem Lanzettenhalter (3) verbundener Steuerzapfen (43) eingreift, wobei mindestens ein Teil der Einstich- und Rückführbewegung durch eine Drehbewegung der Steuerkurve (42) bestimmt wird, bei der der Steuerzapfen (43) die durch die Ausnehmung (41) gebildete Steuerkurve (42) abfährt und wobei die maximale Auslenkung des Lanzettenhalters (3) in Einstichrichtung durch einen unteren Umkehrpunkt (44) der Steuerkurve bestimmt wird.

7. Blutlanzettenvorrichtung nach Anspruch 6, **dadurch gekennzeichnet, daß** die die Steuerkurve (42) bildende Ausnehmung (41) in dem Antriebsrotor (16) ausgebildet ist.

8. Blutlanzettenvorrichtung nach einem der vorhergehenden Ansprüche, **dadurch gekennzeichnet, daß** sie einen Spannhebel (33) aufweist, der zum Spannen des Lanzettenantriebs ein Drehmoment in Drehrichtung (28) auf ein an dem Antriebsrotor (16) vorgesehenes Widerlagerteil (34) ausübt.

9. Blutlanzettenvorrichtung nach einem der vorhergehenden Ansprüche, **dadurch gekennzeichnet, daß** die Druckfläche (23) in der Umgebung des Scheitelpunktes stetig konvex gekrümmt ist, so daß die Spannbewegung ohne Stop in die Einstichbewegung übergeht.

10. Blutlanzettenvorrichtung nach einem der vorhergehenden Ansprüche, **dadurch gekennzeichnet, daß** die Druckfläche bei dem Scheitelpunkt eine Vertiefung (52) aufweist, in die das Druckelement einrastet.

11. Blutlanzettenvorrichtung nach einem der vorhergehenden Ansprüche, **dadurch gekennzeichnet, daß** das Gehäuse ein unteres Abschlußteil (46) mit einer Kontaktfläche (10) aufweist, das eine längliche Form hat, deren Hauptachse parallel zu der Rotorebene verläuft, und das an einer seiner Schmalseiten mittels eines Scharniers (47) schwenkbar an dem übrigen Gehäuse angelenkt ist.

12. Blutlanzettenvorrichtung nach Anspruch 11, **dadurch gekennzeichnet, daß** das Abschlußteil (46) an der dem Scharnier (47) gegenüberliegenden Schmalseite eine Verstellvorrichtung zur Verstellung des Abstandes von dem übrigen Gehäuse und damit zur Einstellung der Einstichtiefe aufweist.

## Claims

1. Blood lancet device (2) for withdrawing blood from a body part (9) for diagnostic purposes, comprising
a lancet holder (3) for holding a lancet (6) and movable within a housing (1) along a predetermined puncturing path,
a lancet guide (13) guiding the lancet holder (3) along the predetermined puncturing path,
a lancet drive (5) with a drive rotor (16) driven by a drive spring (17) in a sense of rotation (28), by which lancet drive the relaxation movement of the drive spring (17) is converted into a pricking movement during which the lancet (6) held by the lancet holder (3) moves with high speed along the predetermined puncturing path in a pricking direction (14) to perform the pricking and retraction movement required to generate a wound in the body part (9),
wherein
the drive rotor has a pressure surface (23) which is directed radially outwardly and runs with varying center distance around the rotation axis (15) in such a manner that it has a vertex (27) with maximum center distance, and a propelling section (30) following the vertex (27) against the sense of rotation (28), with a center distance decreasing against the sense of rotation (28),
the drive spring (17) is adapted for effecting in a propelling angle section of the drive rotor (16) via a pressure element (24) a pressure with a component in radial direction to the rotation axis (15) onto the propelling section (30) of the pressure surface (23), thus driving the drive rotor (16) by the pressure of the drive spring (17) onto the pressure surface in the propelling section (30) in the sense of rotation (28),
the drive rotor (16) is coupled via an output-side coupling mechanism with the lancet holder (3) in such a manner that the maximum displacement of the lancet (6) is reached while the drive rotor (16) is in the propelling angle section, and
the pressure surface (23) comprises a tensioning section (31) preceding the vertex (27) against the sense of rotation (28), and having an increasing center distance against the sense of rotation, the pressure element (24) moving along the tensioning section (31) during tensioning of the lancet drive (5).

2. Blood lancet device according to claim 1, **characterized in that** the propelling section (30) has a convex shape in at least a partial section thereof.

3. Blood lancet device according to any one of the preceding claims, **characterized in that** the tensioning section (31) has a convex shape in at least a partial section thereof.

4. Blood lancet device according to any one of the preceding claims, **characterized in that** the pressure surface (23) has at least two vertexes (27).

5. Blood lancet device according to any one of the preceding claims, **characterized in that** the pressure surface (23) is a continuous surface completely surrounding the axis.

6. Blood lancet device according to any one of the preceding claims, **characterized in that** the output-side coupling mechanism includes a recess (41), rotatable together with the drive rotor, and forming a control curve (42), the recess (41) meshing with a traveler (43) which is linked to the lancet holder (3), whereby at least a part of the pricking and retraction movement is determined by a rotation movement of the control curve (42), during which the traveler (43) moves along the control curve (42) formed by the recess (41), the maximum displacement of the lancet holder (3) in pricking direction being determined by a lower reverse point (44) in the control curve.

7. Blood lancet device according to claim 6, **characterized in that** the recess (41) which forms the control curve (42) is formed in the drive rotor (16).

8. Blood lancet device according to any one of the preceding claims, **characterized in that** it comprises a tensioning lever (33) which effects, for the tensioning of the lancet drive, a torque in the sense of rotation (28) onto a thrust piece (34) of the drive rotor (16).

9. Blood lancet device according to any one of the preceding claims, **characterized in that** the pressure surface (23) has in its range adjacent the vertex a continuous convex shape, so that the tensioning movement passes over to the pricking movement without a stop.

10. Blood lancet device according to any one of the preceding claims, **characterized in that** the pressure surface has a recess (52) at the vertex, where the pressure element latches in.

11. Blood lancet device according to any one of the preceding claims, **characterized in that** the housing has an end piece (46) with a contact surface (10), the end piece has an elongated shape, the main axis of which runs parallel to the rotor plane, and the end piece is articulated with a hinge (47) to the housing on one of its shorter sides.

12. Blood lancet device according to claim 11, **characterized in that** the end piece (46) is provided with an adjusting device at the shorter side opposite to the hinge (47), for the adjustment of the distance from the housing and thus for adjusting the pricking depth.

## Revendications

1. Dispositif de prélèvement de sang par lancette (2) servant au prélèvement de sang sur une partie du corps (9), à des fins de diagnostic, comprenant :
- un support de lancette (3) mobile dans un boîtier (1) le long d'une trajectoire de ponction prédéterminée et servant à maintenir une lancette (6),
- un guidage de lancette (13) par lequel le support de lancette (3) est guidé sur la trajectoire de ponction prédéterminée, et
- un entraînement de lancette (5) comprenant un rotor d'entraînement (16) actionné par un ressort de commande (17) et pouvant tourner dans une direction de rotation (28), rotor d'entraînement par lequel le mouvement de détente du ressort de commande (17) est transformé en un mouvement de ponction au cours duquel la lancette (6) maintenue par le support de lancette (3) est déplacée à grande vitesse le long de la trajectoire de ponction prédéterminée, dans le sens de ponction (14), pour effectuer le mouvement de ponction et de retour nécessaire à la formation d'une plaie dans la partie du corps (9),
où
- le rotor d'entraînement présente une surface de pression (23) dirigée, vers l'extérieur dans le sens radial, laquelle surface de pression s'étend tout autour de l'axe de rotation (15), suivant un entraxe variable, de manière telle que la surface de pression présente un point culminant (27) avec l'entraxe maximum et, avec un entraxe diminuant dans le sens inverse de la direction de rotation (28), présente une partie poussée en avant (30) qui suit le point culminant (27) dans le sens inverse de la direction de rotation (28),
- le ressort de commande (17) est conçu de manière telle, que dans une plage angulaire de rotation du mouvement de poussée en avant du rotor d'entraînement (16), le ressort de commande, via un élément de pression (24), exerce une pression sur la partie poussée en avant (30) de la surface de pression (23), avec une composante dirigée, dans le sens radial, en direction de l'axe de rotation (15), de sorte que le rotor d'entraînement (16), sous l'effet de la pression du ressort de commande (17) sur la surface de pression, est entraîné dans la partie poussée en avant (30) suivant la direction de rotation (28),
- le rotor d'entraînement (16) est couplé au support de lancette (3), via un mécanisme de couplage monté côté sortie, de manière telle que le déplacement maximum de la lancette (6) soit atteint, tandis que le rotor d'entraînement (16) se trouve dans la plage angulaire de rotation du mouvement de poussée en avant, et
- la surface de pression (23) présente, lorsque l'entraxe augmente dans le sens inverse de la direction de rotation, une partie de tension (31) qui précède le point culminant (27) dans le sens inverse de la direction de rotation (28), lequel entraxe, lors de la tension de l'entraînement de lancette (5), est déplacé par l'élément de pression (24).

2. Dispositif de prélèvement de sang par lancette selon la revendication 1, **caractérisé en ce que** la partie poussée en avant (30) est au moins partiellement courbe de façon convexe.

3. Dispositif de prélèvement de sang par lancette selon l'une quelconque des revendications précédentes, **caractérisé en ce que** la partie de tension (31) est au moins partiellement courbe de façon convexe.

4. Dispositif de prélèvement de sang par lancette selon l'une quelconque des revendications précédentes, **caractérisé en ce que** la surface de pression (23) présente au moins deux points culminants (27).

5. Dispositif de prélèvement de sang par lancette selon l'une quelconque des revendications précédentes, **caractérisé en ce que** la surface de pression (23) est une surface toujours continue entourant complètement l'axe.

6. Dispositif de prélèvement de sang par lancette selon l'une quelconque des revendications précédentes, **caractérisé en ce que** le mécanisme de couplage monté côté sortie comprend un creux (41) pouvant tourner en même temps que le rotor d'entraînement et formant une came de commande (42), creux dans lequel s'engage un tourillon de commande (43) relié au support de lancette (3), où au moins une partie du mouvement de ponction et de retour est déterminée par un mouvement de rotation de la came de commande (42), mouvement de rotation au cours duquel le tourillon de commande (43) descend la came de commande (42) formée par le creux (41) et où le déplacement maximum du support de lancette (3) est déterminé, dans le sens de ponction, par un point d'inversion inférieur (44) de la came de commande.

7. Dispositif de prélèvement de sang par lancette selon la revendication 6, **caractérisé en ce que** le creux (41) formant la came de commande (42) est conformé dans le rotor d'entraînement (16).

8. Dispositif de prélèvement de sang par lancette selon l'une quelconque des revendications précédentes, **caractérisé en ce qu'**il présente un levier tendeur (33) qui, pour la tension de l'entraînement de la lancette, exerce un moment de rotation, suivant la direction de rotation (28), sur une pièce (34) formant contre-palier et prévue sur le rotor d'entraînement (16).

9. Dispositif de prélèvement de sang par lancette selon l'une quelconque des revendications précédentes, **caractérisé en ce que** la surface de pression (23) est courbe de façon toujours convexe, autour du point culminant, de sorte que le mouvement de tension se transforme, sans interruption, en mouvement de ponction.

10. Dispositif de prélèvement de sang par lancette selon l'une quelconque des revendications précédentes, **caractérisé en ce que** la surface de pression présente, au niveau du point culminant, une cavité (52) dans laquelle s'engage l'élément de pression.

11. Dispositif de prélèvement de sang par lancette selon l'une quelconque des revendications précédentes, **caractérisé en ce que** le boîtier présente une partie inférieure de protection (46) avec une surface de contact (10), laquelle partie inférieure de protection a une forme allongée dont l'axe principal s'étend parallèlement au plan du rotor et qui, au niveau de l'un de ses petits côtés, est articulée au moyen d'une charnière (47) en pouvant pivoter sur l'autre partie du boîtier.

12. Dispositif de prélèvement de sang par lancette selon la revendication 11, **caractérisé en ce que** la partie de protection (46) présente, sur le petit côté placé à l'opposé de la charnière; (47), un dispositif de réglage permettant de régler la distance par rapport à l'autre partie du boîtier et, par conséquent, de régler la profondeur de ponction.
